# EUROPEAN PATENT SPECIFICATION

(11) **EP 1 887 083 B1**
(45) Date of publication and mention of the grant of the patent: **21.12.2011**
(21) Application number: 06425576.3
(22) Date of filing: 07.08.2006
(51) Int. Cl.: C12N 15/63

(54) **Use of the 5'UTR region of the ZAM retrotrasposon of D. melanogaster as "insulator" to guarantee transgene high expression levels**
Verwendung der 5'UTR des D.melanogaster ZAM Retrotransposons als "Isolator" um eine hohe Transgen-Expression zu garantieren
Utilisation de la region 5'UTR du transposons ZAM de D. melanogaster comme insulateur pour garantir l'expression élevé de transgènes

(43) Date of publication of application: 13.02.2008
(73) Proprietor: Universita' degli Studi di Bari, 70121 Bari (IT)
(72) Inventor: Viggiano, Luigi, 70100 Bari (IT); Minervini, Crescenzio, 70100 Bari (IT); D'Aiuto, Leonardo, 70100 Bari (IT)
(74) Representative: Trupiano, Federica

(56) References cited:
- WO-A2-02/22839
- WO-A2-2004/065581
- CONTE CAROLINE ET AL: "Coupling of enhancer and insulator properties identified in two retrotransposons modulates their mutagenic impact on nearby genes" March 2002 (2002-03), MOLECULAR AND CELLULAR BIOLOGY, VOL. 22, NR. 6, PAGE(S) 1767-1777 , XP002408673 ISSN: 0270-7306 * the whole document *
- LEBLANC P ET AL: "Invertebrate retroviruses: ZAM a new candidate in D. melanogaster" EMBO JOURNAL, OXFORD UNIVERSITY PRESS, SURREY, GB, vol. 24, no. 16, 15 December 1997 (1997-12-15), pages 7521-7531, XP002076117 ISSN: 0261-4189

## Description

The present invention relates to the use of the 5'UTR region of the transposable element of class I ZAM of *Drosophila melanogaster* as "barrier element" or "insulator" for the treatment of genetic diseases. More specifically, the invention concerns the application of a portion of 700 bp (base pairs) of the 5'UTR region of ZAM in the design and production of viral vectors capable of guaranteeing high levels of expression of the transgenes transported into the target cells during treatment of monofactorial genetic diseases.

The invention also relates to vectors comprising said sequence as insulator and their use to prepare medicaments intended for gene therapy.

As it is known, gene therapy consists in inserting genetic material into the cells of the patient to be treated in order to restore cellular function endangered by the absence or malfunctioning of genes. Although there are still a great many technical problems to be solved, ever since its first formulation the unlimited potentials of this new type of therapy have been apparent. In fact, all biological processes are regulated by genes and can therefore be corrected in case of need by the introduction and correct expression of new copies of the genes (transgenes).

Both the genome project (which allowed an exponential increase in knowledge of the genetic bases of diseases) and scientific and economic interests regarding this new form of medicine underlie the growing interest in developing effective protocols for gene therapy.

Among the vectors utilized in gene therapy, viral vectors have a dominating position and those deriving from retroviruses and lentiviruses are the majority.

These last named are integration vectors and this should guarantee long-term expression with quantitatively acceptable levels of the transgene. In fact, these vectors are the most widely utilized for delivery, or entry into the correct cell compartment, of transgenes to the haematopoietic stem cells. This type of stem cells give rise to all types of haematic cells and, consequently, they are the target cells of gene therapy in blood diseases.

Unfortunately, the use of retroviral and lentiviral vectors still have some problems that reduce their effectiveness:
1) the viral sequences are extremely susceptible to transcriptional silencing due both direct methylation of their DNA and to the influence of the regions surrounding the integration site of the vector;
2) moreover, the regulatory sequences (enhancer and silencer) present in the regions surrounding the integration site of the vector can in turn influence transgene expression.

This type of phenomena, which take the name of "position effect", limits correct regulation and expression of introduced transgenes.

It is evident that any gene therapy protocol involving delivery of a transgene must overcome the aforesaid problems. Even when the transgene is carried with the regulatory region thereof, it is still not guaranteed that the level of transgene expression will be sufficient to ensure the therapeutic effect. For example, in the case of thalassemias, notwithstanding the fact that the transgene is carried with the regulatory regions (LCR) thereof, the level of protein produced is still subtherapeutic and, moreover, is subject to further reduction in the course of time.

In view of all this, a system which is capable of ensuring a high and long-term level of expression of a transgene carried with integrating viral vectors is extremely precious.

For this reason, in gene therapy it is fundamental to use sequences capable of isolating the transcriptional unit of the transgene from the various types of position effect described previously.

This type of sequences takes the name of "barrier elements" or "insulators".

Insulators are cis-acting elements that separate the various functional domains of the genes from one another along the eukaryotic chromosome.

Insulators can be operatively defined as sequences that, when placed between an enhancer and its promoter, are capable of blocking interaction thereof (enhancer-blocking action) and capable of effectively combating the silencing and heterochromatization phenomena responsible for the position effect to which all transgenes are subject (barrier action).

The barrier function of an insulator can protect a chromatin domain from non-specific effects of the surrounding genome by blocking the passage of regulatory signals from adjacent loci and chromatin domains.

Due to these characteristics, the use of this type of sequences to construct retroviral and lentiviral vectors for gene therapy is fundamental. They are cloned upstream and downstream of the transgene and of its promoter and in this position are capable of blocking all the position effects that could invalidate correct expression of the therapeutic gene (barrier effect).

Currently, very few insulator elements are known (about twenty) and of these only a small part have been molecularly characterized.

These include some Drosophila melanogaster insulators, such as the scs and scs' elements and the Su(Hw) insulator of the 5'-UTR region of the *gypsy* retrotransposon, which hereinafter we shall call I_{gy}. In vertebrates we find the HS4 insulator of the Locus Control Region (LCR) of the beta globin cluster.

The HS4 insulator has already been utilized to construct retroviral vectors for gene therapy and its presence is capable of increasing the fraction of transduced cells that express the transgene integrated into the chromatin.

In any case, in some integration sites HS4 has a silencing effect on transgene transcription, while at other times the insulator effect is only found in some types of stem cells and not in others.

These limitations make the use of HS4 somewhat complicated and direct research towards the discovery and characterization of new insulators that are capable of overcoming these problems allowing improvement of the performance of vectors.

The difficulty in identifying new insulators is due to the fact that the sequences thereof are not homologous with one another and that there are no genetic tests that detect their presence.

It has now be found that a sequence of 700 base pairs in the 5'-UTR region of the retrotransposon of *Drosophila melanogaster* "ZAM", organized in repeats, hereinafter defined "Z3 sequence" or also merely "Z3", has the characteristics of insulator and is capable of very effectively isolating the transgene with which it is coupled from the effects of the surrounding elements.

The Z3 sequence, GenBank Accession Number: AJ000387, is described in Leblanc P., Desset S., Dastugue B., Vaury C. "Invertebrate retroviruses: ZAM a new candidate in D.melanogaster." EMBO J 16(24), 7521-7531 (1997).

Therefore, according to one aspect thereof, the invention relates to the use of the 700 base pairs in the 5'-UTR region of the retrotransposon of Drosophila melanogaster nucleotide sequence, hereinafter called "Z3" sequence, or to a sequence having a homology of at least 80% with said Z3 sequence, as insulator.

"Sequence having a homology of at least 80% with the Z3 sequence" is intended as a fragment or a variant of the Z3 sequence having at least a homology of 80% with said Z3 sequence, i.e. 90% or 95% of homology.

According to another aspect thereof, the invention relates to the use of the Z3 sequence to isolate a transgene from the position effects and more specifically to increase the expression of a transgene in a cell, *in vitro* or *in vivo*.

According to another aspect thereof, the invention relates to the use of the Z3 sequence to prepare a recombinant expression vector.

Recombinant expression vectors comprising at least one Z3 sequence as insulator are a further object of the invention.

According to another aspect, the invention relates to the use of a recombinant expression vector comprising the Z3 sequence, for the preparation of a medicament and/or of a pharmaceutical composition intended for gene therapy.

Medicaments and pharmaceutical compositions comprising the recombinant expression vector of the invention form a further aspect of the invention, possibly in combination with at least one pharmaceutically acceptable carrier.

According to another aspect thereof, the invention relates to the use of a recombinant expression vector comprising at least two of the Z3 sequence in the production of proteins.

According to another aspect thereof, the invention relates to a host cell transformed with a recombinant expression vector comprising the Z3 sequence.

The invention also concerns a method to isolate a transgene from position effects, in particular a method to increase the expression of a transgene comprising coupling the Z3 sequence with said transgene.

According to the invention, viral vectors can be utilized preferably, although not only, retroviral and lentiviral vectors, in general all "integration" vectors, i.e. those in which the DNA must integrate into the host genome and which are therefore influenced by the position effect.

These vectors have any therapeutic gene and regulatory regions, homologous or heterologous, flanked, preferably at both ends, by two nucleotide sequences.

The transgene of the invention can be any gene to be transfected.

Advantageously, the transgene is coupled with two Z3 sequences, positioned respectively upstream and downstream of the gene and its promoter.

Preferably, the transgene is a gene intended for treating monofactorial genetic diseases.

Alternatively, the transgene is a gene intended to produce protein for industrial use, such as proteins utilizable as medicaments.

According to the present invention, "position effects" are intended to indicate the influence on transgene expression by gene elements adjacent or close thereto. A particular and common position effect is, for example, transgene silencing; this effect can be eliminated by the presence of the insulator, which thus allows stable and conspicuous expression of the gene into which it is inserted.

Transformed host cells according to the present invention are preferably animal cells, i.e. from mammals, preferably humans. Examples of cells that can be transformed according to the invention include stem cells and somatic cells.

Nonetheless, cells of other type, such as bacterial cells or vegetable cells, can also be transformed with the vectors of the invention.

The mechanism for function of insulator elements has not yet been fully determined, although in this regard various models have been proposed; these are ascribable to two large interpretative trends.

According to the first "model" the insulator sequences interact with a series of proteins which, by positioning themselves on the DNA filament "physically" prevent diffusion of heterochromatin proteins along the double helix.

According to a second model, these sequences are instead capable of interacting, directly or indirectly, with Histone-acetylases (H-Ac). These enzymes are capable of acetylating the histones and this makes the surrounding DNA portion more easily accessible to transcriptional factors and consequently it opposes the phenomenon of heterochromatization.

In any case, irrespective of their action mechanism, it has now been determined that the insulators are capable of protecting any type of gene from the negative effects that the surrounding chromatin is capable of exerting thereon.

The specific characteristic of vectors with the Z3 insulator flanking the gene of interest consists in their different and improved performance with respect to those shown and documented by other insulator elements obtained both from vertebrates and from invertebrates.

The differences in performance are due to the fact that this insulator is capable of interacting with a completely new set of chromatin proteins compared to the set identified for other insulators. In fact, the proteins that interact with Z3 (HP1 and Mod(mdg4)) are proteins which extensive scientific experience has proved to be greatly involved in the organization of chromatin.

All this results in an improvement of the performance shown by the vectors presenting Z3 compared to the same type of vector containing other insulators.

Viral vectors that integrate, derived mainly from retroviruses, have been utilized for over 10 years in clinical trials in the attempt to obtain stable gene transfers in proliferating cells like stem cells. According to prevailing dogma, the retroviral genome and therefore the genome of retroviral vectors integrates randomly into the host genome, so that the risks of the insertion of retroviral genomes causing transformation of a coding sequence are 10⁻¹⁰ (1 out of 10 million non-transforming insertions), and therefore even if more than 10¹⁰ cells are usually transduced in "ex vivo" trials with retroviral vectors, the risk of inducing cancer in the transduced cells and then in the host organism was considered sufficiently low (a series of mutations are required to induce neoplastic transformation) to be acceptable.

This point of view was further reinforced by the fact in hundreds of subjects of clinical trials no kind of problem has ever been revealed.

Recent evidence, obtained from a certain number of studies, is changing the perception of the risks of using retroviral vectors for certain types of gene therapy. In fact, it has been proven that retroviral vectors induce lymphoproliferative disorders and it has been proven that retroviral integrations are less random than was previously thought. Analysis of hundreds of HIV integrations has in fact made it possible to show that the retrovirus preferably integrates into transcriptionally active genes, rather than into the non-coding regions of chromatin.

To solve the problem of insertional mutagenesis, various strategies have been proposed, but it is important to consider the performance of the vectors utilized. In fact, the use of currently available retroviral vectors, which are not able to guarantee therapeutic expression of the gene carried thereby for long periods, unavoidably leads to the need to periodically repeat the treatment.

This is very stressful for the patient, as the procedure to obtain haematopoietic stem cells is long and invasive, and implies an increase in the costs of the entire treatment and above all the risk of causing the phenomenon of insertional mutagenesis which can occur for each new treatment cycle.

Not only does the use of Z3 to improve the performance of these vectors, therefore, have no additional risks, but it also makes this type of therapy safer and makes it possible to decrease the costs by eliminating the need to carry out several treatment cycles.

### DESCRIPTION OF THE FIGURES

Figure 1 shows the position of the Z3 sequence in the ZAM transposable element of *Drosophila melanogaster.*
Figure 2 is a histogram of the MFI (Mean Fluorescent Intensity) values of cells transfected with the constructs with and without insulator.

### EXPERIMENTAL SECTION

### EXAMPLE 1

### Demonstration of the insulator activity of Z3

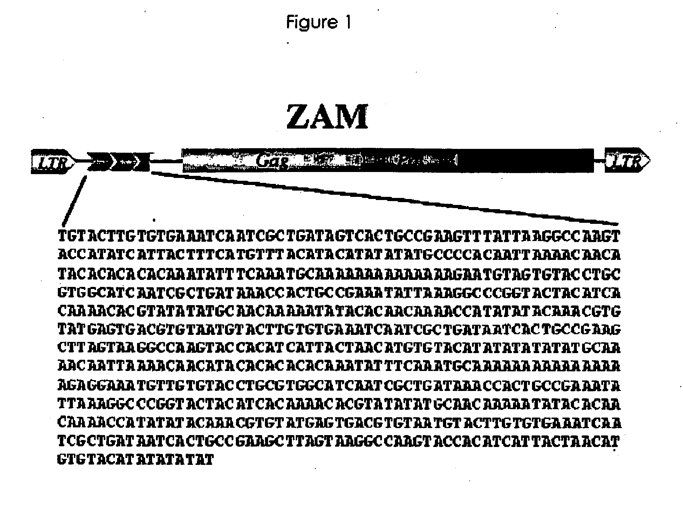

To verify whether Z3, isolated from ZAM of *Drosophila melanogaster* (Figure 1) (GenBank Accession Number: AJ000387) has insulator properties, plasmidic constructs were produced in which Z3 was placed between the enhancer of CytoMegaloVirus (CMV) and its promoter.

All the vectors were obtained by cloning the fragments of DNA of interest in the "promoter-less" vector pGFP-1 (CLONTECH). This vector was designed by Clontech as a tool for the analysis of regulatory sequences.

To compare and validate the results obtained with Z3 we compared these data with those obtained utilizing analogous constructs which however had an insulator tested previously (l_{gy}). In fact l_{gy} works very well in the cells of mammals and in some cases even better than human or mammal insulators (van der Vlag J, den Blaauwen JL, Sewalt RG, van Driel R, Otte AP, J Biol Chem. 2000 Jan 7;275(1):697-704), as is proven by the fact that this sequence is already used to construct vectors for this purpose.

Analysis of the transient expression data obtained by transfecting the human 293 cell line with our constructs confirmed that Z3 behaves as an insulator.

Moreover, by comparing Z3 and I_{gy} it seems that the former is more suitable than the latter to become part of the retroviral vectors for gene therapy as it has greater insulator activity.

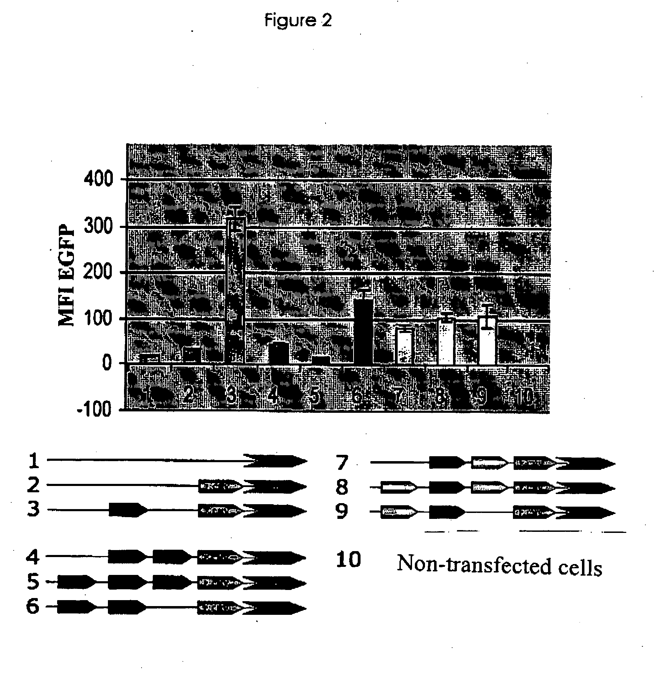

The histogram in Figure 2 indicates the MFI (Mean Fluorescent Intensity) values emitted by the transfected 293 cells, with the constructs indicated at the bottom, which express the gene reporter.

The first three classes (1, 2 and 3) represent the results of the control transfections: the constructs without enhancer show very low levels of GFP gene reporter expression, while the presence of the CMV enhancer guarantees a GFP expression 10 times greater than the one detectable with the construct with the promoter alone. The second triplet of results (4, 5 and 6) instead represent our true experiment, from which it is evident that the presence of Z3 between the enhancer and the promoter is sufficient to reduce GFP expression by at least 6 times and that when the enhancer is completely surrounded by Z3 GFP is expressed at least 20 times less than the control (class 3). Finally, the last triplet allows us to compare the insulator activity of Z3 with that of l_{gy}. As can be clearly seen from classes 7 and 8, the decrease in GFP expression caused by l_{gy} is lower than that highlighted with analogous Z3 constructs.

These results prove that Z3, in human 293 cells, functions as an insulator and does so better than I_{gy}.

## Claims

1. In vitro Use of the 700 base pairs in the 5'-UTR region of the retrotransposon of *Drosophila melanogaster* nucleotide sequence, as disclosed in figure 1, or of a sequence having a homology of at least 80% with said nucleotide sequence, as insulator.

2. Use as claimed in claim 1, to prepare a recombinant expression vector.

3. Use as claimed in claim 1 or 2, to isolate a transgene from position effects.

4. Use as claimed in claim 3, to increase the expression of a transgene.

5. Use as claimed in claims 1 to 4, to produce proteins.

6. Recombinant expression vector comprising at least one transgene and two nucleotide sequences, as defined in claim 1, positioned one upstream and one downstream of said transgene, as insulators.

7. Medicament comprising the recombinant expression vector of claim 6.

8. Host cell transformed with a recombinant expression vector of claim 6.

9. Method to isolate a transgene from position effects comprising coupling at least one sequence, as defined in claim 1, with said transgene.

## Patentansprüche

1. *In-vitro* Verwendung einer 700 Basenpaare langen Nukleotidsequenz aus der 5'-UTR-Region der *Drosophila melanogaster* Retrotransposon-Nukleotidsequenz, wie in Figur 1 offenbart, oder von einer Sequenz, die eine Homologie von zumindest 80 % mit dieser Nukleotidsequenz aufweist, als Isolatorelement.

2. Verwendung gemäß Anspruch 1 zur Herstellung eines rekombinaten Expressionvektors.

3. Verwendung gemäß Anspruch 1 oder 2, um ein Transgen von Positionseffekten zu isolieren.

4. Verwendung gemäß Anspruch 3 zur Erhöhung der Expression eines Transgens.

5. Verwendung gemäß Anspruch 1 bis 4 zur Herstellung von Proteinen.

6. Rekombinanter Expressionvektor enthaltend zumindest ein Transgen und zwei Nukleotidsequenzen wie in Anspruch 1 definiert, wobei eine Nukleotidsequenz stromaufwärts und eine Nukleotidsequenz stromabwärts des Transgens als Isolatorelement positioniert ist.

7. Arzneimittel enthaltend den rekombinanten Expressionvektor gemäß Anspruch 6.

8. Wirtzelle, die mit einem rekombinanten Expressionvektor gemäß Anspruch 6 transformiert ist.

9. Verfahren zur Isolierung eines Transgens gegenüber Positionseffekten enthaltend die Kopplung zumindest einer Sequenz wie in Anspruch 1 definiert mit dem Transgen.

## Revendications

1. Utilisation *in vitro* des 700 paires de bases de la région UTR en 5' du rétrotransposon de la séquence nucléotidique de *Drosophila melanogaster,* telle qu'illustrée sur la figure 1, ou d'une séquence présentant une homologie d'au moins 80 % avec ladite séquence nucléotidique, comme insulateur.

2. Utilisation selon la revendication 1, pour préparer un vecteur d'expression recombinant.

3. Utilisation selon la revendication 1 ou 2, pour isoler un transgène des effets de position.

4. Utilisation selon la revendication 3, pour augmenter l'expression d'un transgène.

5. Utilisation selon les revendications 1 à 4, pour produire des protéines.

6. Vecteur d'expression recombinant comprenant au moins un transgène et deux séquences nucléotidiques, telles que définies dans la revendication 1, une positionnée en amont et une en aval dudit transgène, en tant qu'insulateurs.

7. Médicament comprenant le vecteur d'expression recombinant selon la revendication 6.

8. Cellule hôte transformée par un vecteur d'expression recombinant selon la revendication 6.

9. Procédé permettant d'isoler un transgène des effets de position comprenant le couplage d'au moins une séquence, telle que définie dans la revendication 1, avec ledit transgène.
